# EUROPÄISCHE PATENTANMELDUNG

(11) **EP 4 474 645 A1**
(43) Veröffentlichungstag der Anmeldung: **11.12.2024**
(21) Anmeldenummer: 24179535.0
(22) Anmeldetag: 03.06.2024
(51) Int. Cl.: F04B 43/12, A61M 60/279

(54) **PERISTALTIKPUMPE**

(30) Priorität: 07.06.2023 DE 102023115068
(71) Anmelder: B. Braun Avitum AG, 34212 Melsungen (DE)
(72) Erfinder: JAKOBI, Marco, 37318 Uder (DE); SCHAEFER, Oliver, 36286 Neuenstein (DE); STENZEL, Bruno, 34346 Hann. Münden (DE)
(74) Vertreter: Winter, Brandl - Partnerschaft mbB

(57) **Zusammenfassung**

Peristaltikpumpe, insbesondere zum Fördern von Fluid in einer Vorrichtung zur extrakorporalen Blutbehandlung, mit einem Pumpengehäuse, in dem ein um eine Rotorachse rotierbarer Rotor mit zumindest zwei in Umfangsrichtung zueinander versetzten Quetschelementen aufgenommen ist und welches eine sich bogenförmig um die Rotorachse erstreckende und radial von dem Rotor beabstandete Stützfläche aufweist, die zum Abstützen eines radial zwischen den Rotor und die Stützfläche einbringbaren Schlauchsegments eingerichtet ist. Der Rotor (10) trägt das Quetschelement (20) über eine Schwinge (41), die schwenkbeweglich und abgefedert am Rotor (10) festgelegt ist. Die Schwinge trägt ihrerseits eine Einführhilfe (70), mit der das Schlauchsegment bei dessen Einfädelvorgang in das Pumpenbett drückbar ist, sowie Schlauchführungselemente (70, 72) trägt, mit denen das Schlauchsegment mittig zum Quetschelement (20) ausrichtbar ist. Zur Bereitstellung einer Peristaltikpumpe, die sich bei erweiterter Funktionalität und Lebensdauer durch eine vereinfachte Herstellung auszeichnet, besteht die Schwinge (41) aus hochfestem Edelstahl mit hoher chemischer Beständigkeit und sie ist im Pulverspritzgieß-, vorzugsweise im Metal-Injection-Molding (MIM)-Verfahren hergestellt.

## Beschreibung

Die Erfindung betrifft ein eine Schlauchrollen- bzw. Peristaltikpumpe, d.h. eine Verdrängerpumpe, bei der das zu fördernde Medium durch äußere mechanische Verformung eines Schlauches durch diesen hindurchgedrückt wird, gemäß dem Oberbegriff des Patentanspruchs 1, sowie eine für diese Peristaltikpumpe zugeschnittene Schwinge. Derartige Pumpen werden häufig zum Fördern von Fluid, insbesondere Blut, in einer Vorrichtung zur extrakorporalen Blutbehandlung, insbesondere in einer Dialysemaschine, verwendet. Das Fluid wird dabei mittels der Peristaltikpumpe von einer Niederdruckseite zu einer Hochdruckseite gefördert, wobei eine elastisch verformbare und zwischen der Niederdruckseite und der Hochdruckseite angeordnete Fluidleitung in Form eines Schlauchsegments, welches als Pumpensegment bezeichnet wird, zwischen einer Stützfläche eines Pumpenbetts und einem gegenüber dieser rotierenden Rotor mit zumindest zwei Quetschelementen verformt, insbesondere zusammengequetscht.

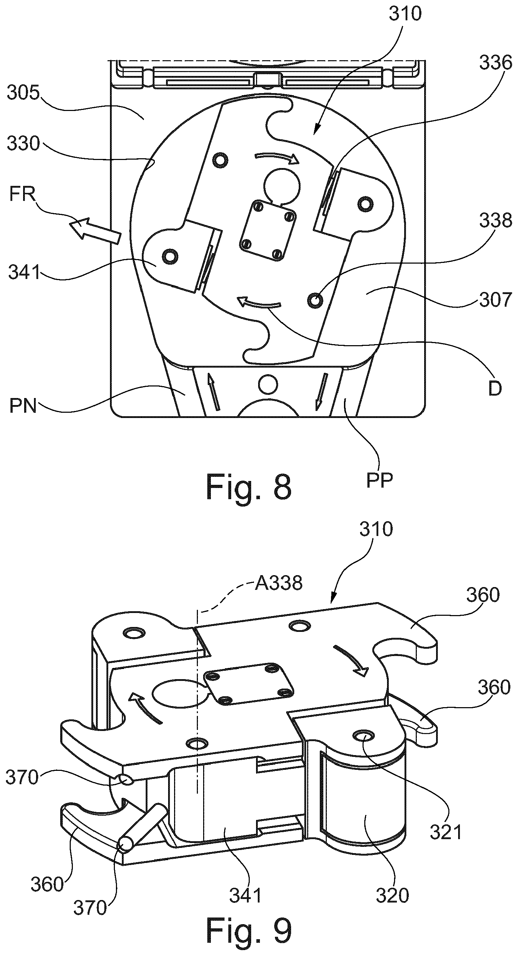

In der Regel sind bei einer Peristaltikpumpe gemäß dem Oberbegriff des Anspruchs 1, wie sie beispielsweise aus dem Dokument EP 1 749 549 B1 bekannt und schematisch in den Figuren 8 und 9 dargestellt ist, am im Pumpengehäuse drehbar gelagerten Rotor zumindest zwei in Umfangsrichtung zueinander versetzt angeordnete Quetschelemente 320 in Form von gefedert gelagerten Andruckrollen vorgesehen. Die Stützfläche 330 wird von einer Kreissegmentfläche gebildet, die sich über einen ausreichend großen Zentriwinkel größer als 180° erstreckt. Die Andruckrollen sitzen mittels einer Rollenachse 321 drehbar gelagert an Schwingen 341, die gelenkig mittels eines Gelenkstifts 338 mit der Achse A338 am Rotor 310 schwenkbeweglich festgelegt und von einer Druckfeder 336 mit einer radial nach außen gerichteten Kraft FR (siehe Figur 8) beaufschlagt sind.

Um die Peristaltikpumpe in Betrieb zu nehmen, ist es erforderlich, ein in Figur 8 nicht näher dargestelltes Schlauchsegment, also das Pumpensegment, von einer niederdruckseitigen Anschlussstelle PN bogenförmig oberhalb des Rotors 310 der Stützfläche 330 folgend zu einer hochdruckseitigen Anschlussstelle PP zu führen und es dann durch manuelles Drehen des Rotors 310 in Richtung der Pfeile D in das Pumpenbett 307 einzufädeln. Als Einfädel- bzw. Einführhilfe zur Unterstützung des manuellen Einfädelns hat die bekannte Peristaltikpumpe zwei vom Rotor 310 getragene Führungsvorsprünge in Form von Haken 360, die zwischen den beiden Andruckrollen 320 angeordnet sind. Abweichend von Konzepten mit automatischer Einfädelung des Pumpensegments, bei denen der Rotor selbsttätig in eine optimale Einfädelposition ausgerichtet wird, muss der Anwender im Fall der aus dem Dokument EP 1 749 549 B1 bekannten Peristaltikpumpe darauf achten, den Schlauch vor dem Haken 360 einzulegen, um ein Verklemmen zu verhindern. Dazu muss der Rotor 310 manuell, wie in der Figur 8 dargestellt ausgerichtet werden. Für die Führung des von den Haken 360 bereits erfassten Schlauchsegments und zu dessen Ausrichtung mittig zum Quetschelement werden zusätzlich in den Rotor eingesetzte, als Schlauchführungselemente dienende Führungsstifte 370 genutzt, die den Haken 360 nachlaufen.

Um den Einfädelvorgang zu vereinfachen, wäre es von Vorteil, die Einführhilfe und/oder die Schlauchführungselemente in die Schwinge zu integrieren. Damit wird allerdings die Form der Schwinge noch komplexer, wodurch sich die Herstellung der Schwinge noch aufwendiger gestaltet.

Bekannte Schwingen von Rotoren bei Blutpumpen sind deshalb entweder Kunststoff-Spritzguss-Teile oder mechanisch gefertigte Teile aus Kunststoff oder Metall.

Weil derartige Schwingen bedingt durch die zyklisch aufzunehmenden Biegekräfte eine hohe Formstabilität und Dauerschwingfestigkeit benötigen, hat die Anmelderin auch bereits versucht, Schwingen mit integrierter Schlauchführung aus Aluminium herzustellen und die Kontur durch ein Fräsverfahren aus dem Vollen zu erzeugen. Selbst wenn die Schlauchführungselemente als gesonderte Stifte ausgebildet werden, muss in diesem Fall sehr viel Material meistens spanend abgetragen werden, so dass der Rohmaterialeinsatz im Vergleich zum Fertigteil sehr hoch wird. Ein weiteres Problem besteht darin, dass die Schwinge für die Befestigung des Quetschelements bzw. der Andruckrolle mit der Rollenachse verbördelt werden muss. Aufgrund der geringen Festigkeit des Aluminiums kann es beim Verbördeln der Rollenachsen zu geringen Verformungen kommen. Um Ausschuss zu vermeiden, ist daher eine genaue Prozessüberwachung erforderlich. Zusätzlich müssen die gefrästen Aluminium-Schwingen noch eloxiert werden, um die chemische Beständigkeit sicher zu stellen. Da sich das Eloxieren auf die Genauigkeit der Passungen in den Bohrungen auswirkt, müssen die Bohrungen vor dem Eloxieren mit Stopfen verschlossen werden. Das Eloxieren und Verstopfen stellen einen zusätzlichen Mehraufwand dar. Bei Verwendung hochfester Werkstoffe wird die mechanische Bearbeitung noch aufwendiger und kostenintensiver.

Der Erfindung liegt die Aufgabe zugrunde, eine gattungsgemäße Peristaltikpumpe zu schaffen, die sich bei erweiterter Funktionalität und Lebensdauer durch eine vereinfachte Herstellung auszeichnet.

Diese Aufgabe wird mit den Merkmalen des Anspruchs 1 gelöst.

Neuerungsgemäß besteht die Schwinge des Rotors aus einem hochfesten Edelstahl mit sehr guten chemischen Eigenschaften, wobei die Schwinge im MIM-Verfahren (Metal Injection Molding) hergestellt ist. Diese Herstellung ermöglicht die Integration zusätzlicher Funktionen, wie Einfädelhilfe und Schlauchsegment-Führung bei gleichzeitiger erheblicher Reduzierung des eingesetzten Rohmaterials und der Herstellkosten im Vergleich zu einer spanenden Fertigung. Dabei kann durch das formgebende MIM-Verfahren die mechanische Nacharbeit auf ein Minimum beschränk bleiben. Zusätzlich können durch das MIM-Verfahren filigrane Konturen (z.B. Verstärkungsrippen) realisiert werden, wodurch die Masse des Bauteils bei gleichbleibender Festigkeit noch weiter reduziert werden kann. Diese filigranen Konturen sind frästechnisch nur schwer oder gar nicht herstellbar. Durch die im MIM-Verfahren erzielbare gute Oberflächenqualität ist im Bereich der mit dem Schlauchsegment in Kontakt kommenden Oberflächen keine spezielle Nacharbeit erforderlich, was sich zusätzlich für die Verringerung der Fertigungskosten nutzen lässt.

Versuche haben gezeigt, dass derartige Werkstoffe, wie etwa ein austenitischer, hitzebeständiger Chrom-Nickel-Stahl, wie er z.B. unter der Bezeichnung 1.4841 (X15CrNiSi25-21) bekannt ist, ohne Weiteres mit ausreichend hohen Dichten im MIM-Verfahren herstellbar sind und die daraus hergestellten Komponenten optimale Eigenschaften für den Einsatz in Peristaltikpumpen haben. Die so hergestellte Schwinge ist gegen verschiedenste Medien chemisch beständig und sie hat bei geringstem Materialeinsatz von nur wenigen cm³ eine so hohe Festigkeit, dass zum einen die Andruckkraft der Rollen im Förderbetrieb der unterschiedlichsten Medien mit graziler Bauart sichergestellt und zum anderen das Verbördeln der Rollenachsen ohne Verformung der Schwingen gewährleistet ist. Dabei ergibt sich der zusätzliche Vorteil, dass die chemischen Eigenschaften des Werkstoffes eine zusätzliche Oberflächenbehandlung wie zum Beispiel ein Lackieren oder Eloxieren ersparen.

Vorteilhafte Weiterbildungen sind Gegenstand der Unteransprüche.

Durch die neuerungsgemäße Auswahl von Material und Herstellungsverfahren wird die Möglichkeit eröffnet, der Schwinge bei kompakter Bauart weitere Funktionen zu übertragen, wie z.B. die Komponenten für die Führung des Schlauchsegments in die Schwinge zu integrieren, und zwar so, dass sie nahe zur Halterung des Quetschelements bzw. der Andruckrolle liegen. Die Schwinge kann so mittels eines die Andruckrolle rahmenartig einfassenden Brückenteils eine beidseitige Lagerstelle für die Andruckrolle ausbilden, wodurch die Kräfte von der Andruckrolle sicher und verformungsarm in die Schwinge eingeleitet werden können.

In den weiteren Unteransprüchen sind Ausgestaltungen der Schwinge festgelegt, mit denen das Betreiben der Pumpe, insbesondere das Handling beim Einfädeln und/oder Ausfädeln und bei der zentrischen Führung des in der Pumpe aufgenommenen Schlauchsegments, also des Pumpensegments, mittig zu den Andruckrollen erleichtert wird. Gleichzeitig wird hierdurch die Form der Schwinge immer komplexer. Während die sich dadurch ergebenden Freiräume der Schwinge bei der spanenden Bearbeitung eines Rohlings durch Materialabtrag geschaffen werden, wird bei der neuerungsgemäßen Ausgestaltung der Schwinge nur das benötigte Material in das Werkzeug eingespritzt. Das fertige Bauteil besitzt beispielsweise nur ein Volumen von ca. 4 cm³, während man für ein aus dem Vollen gefrästes Bauteil ein Halbzeug mit einem Volumen von min. 40 cm³ benötigen würde. Durch das MIM-Verfahren können somit Rohstoffe in erheblichem Umfang gespart werden. Weil mit der neuerungsgemäßen Herstellung der Schwinge auch ohne Weiteres sogar hinterschnittene Rippen herstellbar sind, kann die Masse der Schwingebei gleichbleibender Festigkeit reduziert werden.

Neben herstellungstechnischen Vorteilen ergeben sich mit der neuerungsgemäßen Gestaltung der Schwinge auch Vorteile beim Betreiben der Peristaltikpumpe. Weil sowohl die Einführhilfe als auch die Schlauchführungselemente dem Quetschelement in Drehrichtung des Rotors benachbart in die Schwinge integriert sind, werden die Teilezahl und damit auch die Fertigungs- und Montagekosten der Pumpe verringert, da es hierzu nur noch der Montage der Schwinge bedarf. Weil die Einführhilfen benachbart zu, also jeweils direkt vor dem zugeordneten Quetschelement liegen, wird das manuelle Einlegen des Pumpensegments ermöglicht, ohne dass der Anwender auf eine spezielle Position des Rotors achten muss.

Vorteilhafterweise ist die Schwinge am Rotor mittels eines Schwenklagers angebracht, welches zum Quetschelement in Drehrichtung des Rotors versetzt angeordnet ist, wobei die Einführhilfe und die Schlauchführungselemente am Schwenklager sitzen. Mit dieser Gestaltung kann der Aufbau der Schwinge mit integrierter Einführhilfe und Schlauchführung besonders kompakt gehalten werden.

In Versuchen hat sich gezeigt, dass es aufgrund der neuerungsgemäßen Anordnung der Einführhilfe am Schwenklager der Schwinge zum schonenden und zuverlässigen Einfädeln des Schlauchsegments genügt, die Einführhilfe als einfachen, stiftartigen Leitkörper auszubilden, der sich vom Schwenklager im Wesentlichen in radialer Richtung von der Rotorachse weg in Richtung Stützfläche erstreckt und der im Querschnitt betrachtet im Wesentlichen eine abgerundete, in Drehrichtung der Rotors weisende Keilform hat, die zum Pumpenbett unter einem spitzen Winkel, vorzugsweise zwischen 30 und 50°, angestellt ist.

Dabei ist es weiter vorteilhaft, wenn der Leitkörper zwei Funktionsflächen aufweist, von denen die die in Drehrichtung des Rotors vorlaufende, einer Mittelebene der Schwinge abgewandte äußere Funktionsfläche eine abgerundete Keilfläche ausbildet und die der Mittelebene zugewandte innere Funktionsfläche von einer sich an die Keilfläche vorzugsweise stufenlos anschließenden Zylindersegmentfläche gebildet ist. Die abgerundete Keilfläche drückt das einzufädelnde Schlauchsegment beim manuellen Drehen des Rotors sanft nach unten in das Pumpenbett, wobei die verrundete Geometrie eine Beschädigung des Schlauchsegments verhindert. Die sich an die Keilfläche anschließende Zylindersegmentfläche kann in vorteilhafter Weise zur Führung des Schlauchsegments im Betrieb der Peristaltikpumpe mittig zu den Quetschelementen herangezogen werden. Dabei ist selbstverständlich darauf geachtet, dass die Qualität der mit dem Schlauchsegment in Kontakt kommenden Oberflächen des Leitkörpers so ausgeführt ist, dass das Schlauchsegments bestmöglich geschont wird. Es hat sich gezeigt, dass diese Oberflächenqualität, z.B. eine ausreichend kleine Rauheit, mit dem MIM-Herstellungsverfahren ohne weitere Behandlung der Funktionsflächen erreicht werden kann.

Auch die Maßhaltigkeit des stiftartigen Leitkörpers und dessen Lagezuordnung zu den Lagerachsen ist mit dem MIM-Verfahren sichergestellt, indem der Vorteil der linearen Schrumpfung beim Sinterprozess ausgenützt bzw. durch Probesinterungen genauestens die Schrumpfung vorberechnet wird.

Der Querschnitt des Leitkörpers kann unterschiedlichste Form haben, solange die Keilfläche und die Zylindersegmentfläche auf der dem Schlauchsegment zugewandten Seite anmeldungsgemäß ausgebildet sind. Wenn allerdings der Querschnitt des Leitkörpers eine Mittelachse hat, ergibt sich eine vereinfachte Herstellung insbesondere für den Fall, dass der Leitkörper als separates Bauteil an der Schwinge angebracht wird. Es ist für den alternativen Fall, dass Schwinge und Leitkörper einstückig ausgebildet sind, von zusätzlichem Vorteil, wenn der Leitkörper zusätzlich mittels einer der Mittelachse folgenden Strebe an der Schwinge abgestützt ist. Diese Gestaltung erlaubt es, den Leitkörper noch filigraner und dennoch stabil genug auszubilden.

Wenn der Leitkörper an einem Schwenklagerauge der Schwinge angeordnet, vorzugsweise einstückig an diesem angebracht ist, können die auf den Leitkörper beim Einfädeln aufgebrachten radialen Stützkräfte und Biegekräfte gut in die Schwinge eingeleitet werden, da das Schwenklagerauge von der Schwenkachse stabil abgestützt ist und dementsprechend nur minimalen Verformungen unterliegt.

Eine weitere vorteilhafte Ausgestaltung der Peristaltikpumpe besteht darin, die Schwinge an ihrem dem Schwenklagerauge abgewandten Endabschnitt mit einer Anschlagfläche für eine Druckfeder auszustatten, und das Quetschelement zwischen Schwenklagerauge und Anschlagfläche anzuordnen, beispielsweise drehbar zu lagern. Das MIM-Herstellungsverfahren erlaubt es dabei, die Anschlagfläche optimal zu positionieren, ohne dadurch die Herstellung der Schwinge komplexer zu gestalten. Die Schwinge erhält auf diese Weise eine kompakte Form, so dass am Umfang des Rotors genügend freier Raum für die Ausbildung weiterer Funktionsflächen, wie z.B. von Führungsflächen für das Schlauchsegment verbleibt. Die einzelnen Abschnitte der Schwinge können beliebig dort, wo es auf zusätzliche Festigkeit ankommt, beispielsweise an den Bereichen, die das Schwenklagerauge oder die Drehachse für die Andruckrolle ausbilden, verstärkt werden, wobei der hierfür erforderliche zusätzliche Materialaufwand auf ein Minimum begrenzt bleibt.

Vorzugsweise wird dem die vorstehend beschriebene Einführhilfe ausbildenden ersten Leitkörper ein weiterer, zweiter Leitkörper zugeordnet ist, der bezüglich einer parallel zum Pumpenbett liegenden Horizontalebene symmetrisch zum ersten Leitkörper ausgebildet und orientiert ist. Somit bilden die sich zugewandten Zylindersegmentflächen der beiden spiegelbildlich angeordneten Leitkörper die Funktionsflächen der Schlauchführungselemente aus. Die Integration der Einführhilfe und der Schlauchführungselemente in die Schwinge wird dabei mit einem weiter reduzierten Herstellungsaufwand sichergestellt. Aufgrund der spiegelbildlich angeordneten und ausgerichteten Leitkörper führen diese das Schlauchsegment im Betrieb der Peristaltikpumpe trichterartig und schonend ins mittige Zentrum des betreffenden Quetschelements, was sich positiv auf die Fördergenauigkeit der Peristaltikpumpe auswirkt.

Die Spritzgießform zur Herstellung der Schwinge wird vereinfacht, wenn die Horizontalebene, zu der die beiden Leitkörper symmetrisch angeordnet sind, gleichzeitig eine Symmetrieebene der Schwinge ist.

Grundsätzlich lässt es die Auswahl des Werkstoffs für die Herstellung der Schwinge zu, die Oberflächen der gesinterten Schwinge unbearbeitet zu lassen. Das Schlauchsegment kann allerdings in ausgewählten Fällen zusätzlich vor Abnutzungen geschont werden, wenn die Einführhilfe und vorzugsweise auch die Schlauchführungselemente von einem Werkstoff beschichtet sind, der abriebminimierende Gleiteigenschaften hat.

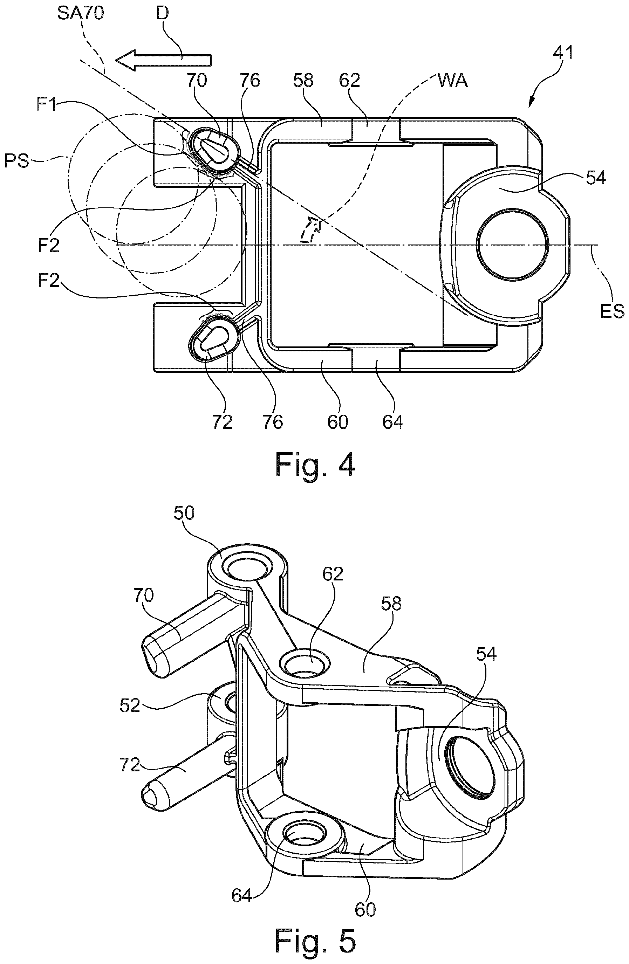

Die Einführhilfe und die Schlauchführungsgeometrie sind gemäß einer vorteilhaften Ausgestaltung in die Schwinge integriert. Dadurch werden Fertigungs- und Montagekosten verringert. Die Schlauchführungsgeometrie besitzt zwei Bereiche. Der in Figur 4 mit strichpunktierter Linie gekennzeichnete Bereich am oberen Leitkörper bzw. Hörnchen ist eine abgerundete schräge Fläche, die beim Einfädeln den Schlauch nach unten drückt. Die verrundete Geometrie verhindert dabei eine Beschädigung des Schlauches. Die in Figur 4 mit gestrichelten Linien gekennzeichneten runden Flächen am oberen und unteren Leitkörper bzw. Hörnchen führen den Schlauch während des Betriebes, so dass das Pumpensegment mittig über die Andruckrollen geführt wird. Die Qualität der Oberflächen ist im Bereich der Schlauchführung so gewählt, dass der Schlauch nicht beschädigt, wird. Die Oberflächenqualität wird ohne speziell Nacharbeit hergestellt. Durch die Anordnung der Einführhilfe direkt vor den Andruckrollen muss der Anwender beim manuellen Einfädeln auf keine spezielle Position des Rotors achten.

Nachstehend werden Ausführungsbeispiele der Erfindung anhand schematischer Zeichnungen näher erläutert. Es zeigen:
Figur 1 eine perspektivische Darstellung einer beispielhaften Ausführungsform einer Peristaltikpumpe, wie sie in Verbindung mit einer Vorrichtung zur extrakorporalen Blutbehandlung Verwendung finden kann;
Figur 2 eine perspektivische Ansicht eines in der Peristaltikpumpe gemäß Figur 1 verwendeten Rotors;
Figur 3 eine perspektivische Ansicht des Rotor-Grundkörpers der Peristaltikpumpe mit angebauter Schwinge;
Figur 4 in stark vergrößertem Maßstab die Seitenansicht der in der Peristaltikpumpe gemäß Figur 1 und 2 verwendeten Schwinge, bei einer Blickrichtung entlang des Pfeils "IV" in Figur 2
Figur 5 eine perspektivische Ansicht der Schwinge;
Figur 6 eine der Figur 5 entsprechende Ansicht der Schwinge mit einem schematisch angedeuteten Werkstoff-Volumen, das für eine Herstellungs-Variante erforderlich wäre;
Figuren 7A und 7B perspektivische Ansichten von durch spanabhebende Bearbeitung hergestellten Schwingen;
Figur 8 eine Draufsicht einer bekannten Peristaltikpumpe; und
Figur 9 eine perspektivische Ansicht des bei der Peristaltikpumpe gemäß Figur 8 verwendeten Rotors.

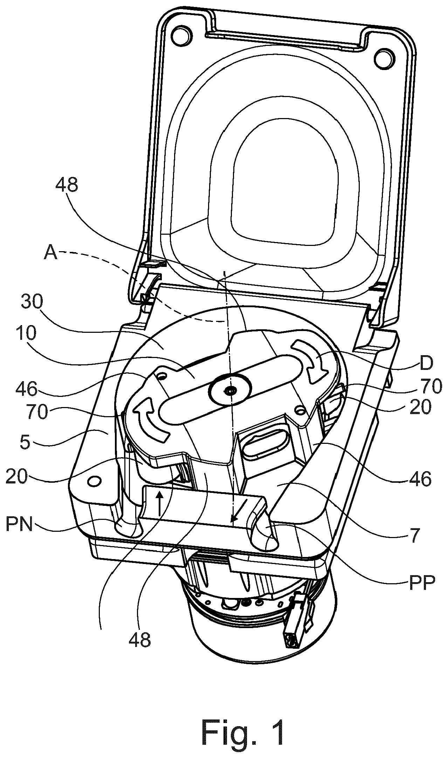

In Figur 1 ist eine Peristaltikpumpe gezeigt, wie sie beispielsweise in Dialysemaschinen eingesetzt wird. Die Peristaltikpumpe hat dort die Aufgabe, ein definiertes Volumen eines Mediums, wie zum Beispiel Blut oder Dialysefluid, durch Verformen und Abklemmen der elastisch verformbaren Fluidleitung zu fördern. Die Peristaltikpumpe zur Förderung von Blut fördert in der Regel von einer negativen Druckseite PN (Niederdruck-Anschlussstelle) auf eine positive Druckseite PP (Hochdruck-Anschlussstelle).

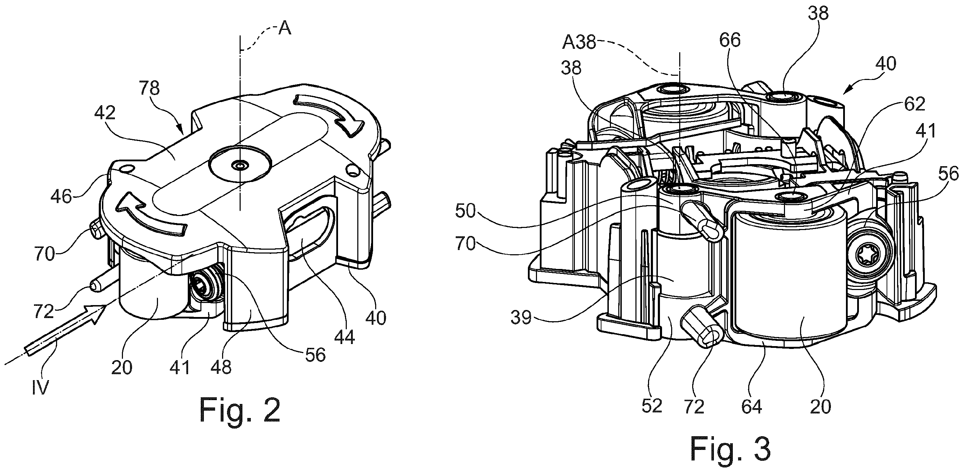

Wie in Figur 1 dargestellt, hat die Peristaltikpumpe ein - in Figur 1 aufgeklappt gezeigtes - Rotor- bzw. Pumpengehäuse 5, in dem ein um eine Rotorachse A rotierbarer Rotor 10 mit zumindest zwei in Umfangsrichtung zueinander beispielsweise diametral versetzten Quetschelementen 20 , hier Druckrollen, aufgenommen ist. Das Pumpengehäuse 5 umfasst ein Pumpenbett 7, welches sich bogenförmig um die Rotorachse A erstreckt und eine radial von dem Rotor 10 beabstandete Stützfläche 30 aufweist. Diese Stützfläche 30 ist zum radialen Abstützen eines radial zwischen ihr und dem Rotor 20 einbringbaren, in den Figuren nicht gezeigten, lediglich in Figur 4 strichpunktiert angedeuteten Pumpensegments PS eingerichtet. Die Quetschelemente 20 sind abgefedert auf einer in Figur 1 nicht näher bezeichneten Schwinge gelagert, die ihrerseits schwenkbeweglich über einen Gelenkstift 38 (siehe Figur 3) am Rotor 10 befestigt ist. Die Quetschelemente 20 sind drehbar auf einer gelenkig am Rotor-Grundkörper 40 federnd abgestützten Schwinge 41 gelagert.

Das in der Peristaltikpumpe eingesetzte Schlauchsegment wird nachfolgend als Pumpensegment bezeichnet. Das in Figur 1 nicht gezeigte Schlauchsegment stützt sich nicht auf einem mit dem Bezugszeichen 7 versehenen Pumpenbett ab, sondern es wird durch weiter unten näher beschriebene Schlauchführungselemente mittig zu den Quetschelementen positioniert. Die Stützfläche 30 ist in der Regel von einer Zylinderfläche gebildet, sie kann jedoch auch muldenförmig vertieft sein. Die Drehrichtung des Rotors 10 beim Fördern von Fluid ist mit dem Pfeil D bezeichnet.

Figur 2 zeigt Einzelheiten des Rotors 10. Er hat einen in Figur 3 perspektivisch gezeigten Rotor-Grundkörper 40 und eine Rotorabdeckung 42. Der Rotor 10 ist durch eine seitlich bedienbare Taste 44 von einer nicht näher gezeigten Antriebswelle abnehmbar.

Die sich mit dem Rotor 10 mitdrehende Rotorabdeckung 42 trägt im Bereich der Quetschelemente 20 Führungsflächen 46, 48, von denen die eine Führungsfläche 46 dem Quetschelement 20 vorläuft und die andere Führungsfläche 48 dem Quetschelement 20 nachläuft. Die Führungsflächen 46, 48 sind demnach jeweils zu beiden Seiten des Quetschelements 20 in Umfangsrichtung benachbart derart angeordnet und gestaltet, dass sie mit der Stützfläche 30 jeweils einen kreissegmentartigen Führungskanal ausbilden, in dem ein in das Pumpenbett 7 eingelegtes Pumpensegment mit vorgegebener Spielpassung radial gefangen ist. Das Pumpensegment kann im Betrieb der Peristaltikpumpe am Pumpengehäuse 5 mittels nicht näher dargestellter Passkörper fixiert sein, die in entsprechenden Anschlüssen des Pumpengehäuses 5 verschiebesicher festlegbar sind.

Bevor die Peristaltikpumpe in Betrieb genommen wird, muss ein Schlauchsegment, das im Betrieb der Pumpe zum Pumpensegment wird, in das Pumpenbett 7 eingeführt bzw. eingesetzt und mittig bezüglich der Quetschelement 20 positioniert werden.

Dazu wird das nicht gezeigte Pumpensegment zunächst- so in das Pumpengehäuse 5 gelegt, dass es sich von der niederdruckseitigen Anschlussstelle PN bogenförmig oberhalb des Rotors 10 der Stützfläche 30 folgend zu einer hochdruckseitigen Anschlussstelle PP erstreckt. Das Pumpensegment PS (in Figur 4 mit strichpunktierter Linie angedeutet) liegt in dieser Phase noch zur Gänze oberhalb des Rotors 10, genauer gesagt oberhalb der Rotorabdeckung 42. Um jetzt das Pumpensegment durch manuelles Drehen des Rotors 10 in Richtung des Pfeils D in das Pumpenbett 7 einzufädeln und dabei die Beanspruchungen des Pumpensegments möglichst gering und die Handgriffe möglichst einfach zu halten, trägt der Rotor 10 eine Einführhilfe, mit der das Schlauch- bzw. Pumpensegment bei dessen Einfädelvorgang in das Pumpenbett 7 drückbar ist, sowie Schlauchführungselemente, mit denen das Schlauch- bzw. Pumpensegment mittig zu den Quetschelementen 20 ausgerichtet werden kann.

Die Einführhilfe und die Schlauchführungselemente sind neuerungsgemäß in die Schwinge 41 integriert, was nachfolgend unter Bezug auf die Figuren nachfolgend anhand der Figuren 3 bis 5 näher beschrieben wird.

Träger der Einführhilfe und der Schlauchführungselemente ist bei der Neuerung die am Rotor-Grundkörper 40 schwenkbeweglich angebrachte Schwinge 41. Genauer gesagt, sind Einführhilfe und Schlauchführungselemente in die Schwinge 41 wie folgt integriert:
Wie in der Figur 3 dargestellt, die den Rotor-Grundkörper 40 zeigt, von dem die Rotorabdeckung 42 abgenommen ist, ist die Schwinge 41 mit der Schwenkachse A38 mittels zweier Schwenklageraugen 50, 52 schwenkbar am Gelenkstift 38 festgelegt. Die Schwinge 41 hat - wie sich den Figuren 3 bis 6 entnehmen lässt - an ihrem den Schwenklageraugen 50, 52 abgewandten Endabschnitt eine Anschlagfläche 54 für eine Druckfeder 56, mit der die Schwinge 41 mit einer vom Rotor-Grundkörper 40 weg gerichteten Druckkraft beaufschlagt wird. Zwischen den Schwenklageraugen 50, 52 und der Anschlagfläche 54 ist die Schwinge 41, wie am besten aus der Darstellung gemäß Figur 5 erkennbar, in Form eines leicht konvex gebogenen Bügels mit zwei im Parallelabstand liegenden Bügelarmen 58, 60 ausbildet, die ihrerseits Lagerstellen 62, 64 für eine Drehachse 66 (siehe Figur 3) der Andruckrolle 20 ausbilden. Die Schwinge 41 hat eine Symmetrieebene ES, zu der auf diese Weise auch die Andruckrolle 20 zentrisch positioniert ist.

Als Einführhilfe und als Führungselemente für das Pumpensegment PS dienen am Schwenklager, genauer gesagt an den Lageraugen 50, 52 angeformte, also in die Schwinge 41 möglichst nahe zu den Andruckrollen 20 integrierte Leitkörper 70, 72, die sich vom Schwenklager, also von der Achse A38 des Gelenkstifts 38 im Wesentlichen in radialer Richtung von der Rotorachse A, also vom Rotor-Grundkörper 40 so weit weg erstrecken, dass sie - wie aus Figur 1 erkennbar, in der allerdings nur ein oberer Leitkörper 70 sichtbar ist - bei Drehung des Rotors 10 der Stützfläche 30 möglichst nahe kommen.

Der obere Leitkörper 70 bildet dabei die weiter unten in ihrer Funktion näher zu beschreibende Einführhilfe, und beide Leitkörper 70, 72 zusammen fungieren als Schlauchführungselemente, mit denen das Pumpensegment mittig zu den Quetschelementen bzw. Andruckrollen 20 ausgerichtet wird.

Zumindest der die Einführhilfe ausbildende obere Leitkörper 70, im gezeigten Ausführungsbeispiel allerdings beide Leitkörper 70, 72, haben im Querschnitt und in der radialen Ansicht gemäß Figur 4 betrachtet im Wesentlichen eine abgerundete, in Drehrichtung D des Rotors weisende Keilform, die zum Pumpenbett 7 und damit zu der dazu parallelen Symmetrieebene ES der Schwinge 41 unter einem spitzen Winkel WA (siehe Figur 4) angestellt ist.

Zumindest der obere Leitkörper 70 hat zwei Funktionsflächen F1 und F2, von denen die in Drehrichtung D vorlaufende, der Symmetrieebene ES abgewandte äußere Funktionsfläche F1, die in Figur 4 mit strichpunktierter Linie markiert ist, eine abgerundete Keilfläche ausbildet und die der Symmetrieebene ES zugewandte innere Funktionsfläche F2, die in Figur 4 mit gestrichelter Linie markiert ist, von einer sich an die Keilfläche vorzugsweise stufenlos anschließenden Zylindersegmentfläche gebildet ist. Der untere Leitkörper 72 bildet zumindest die innere Funktionsfläche F2 aus. Die Funktionsfläche F1 fungiert dabei als Einführhilfe beim Einfädeln des Pumpensegments PS, und die sich gegenüberliegenden Funktionsflächen F2 als Schlauchführungselemente, um das Pumpensegment im Betrieb der Peristaltikpumpe mittig zu den Quetschelementen, d.h. den Andruckrollen 20 zu halten.

Im gezeigten Ausführungsbeispiel hat der Querschnitt der Leitkörper 70, 72 jeweils eine Mittelachse bzw. Symmetrieachse, wobei in Figur 4 nur die Symmetrieachse SA70 des Leitkörpers 70 eingetragen ist. Figur 4 lässt ferner erkennen, dass die Leitkörper 70, 72 zusätzlich mittels einer der Symmetrieachse SA70 folgenden Strebe 76 an der Schwinge 41 abgestützt ist.

Zusammenfassend bildet also der obere Leitkörper 70 mit der äußeren Funktionsfläche F1 die Einführhilfe für das Pumpensegment PS aus, während beide Leitkörper 70, 72 zusammen mit ihren Funktionsflächen F2 als Schlauchführungselemente dienen.

Mit dieser Gestaltung ergibt sich folgende Funktionsweise:
Ausgehend von einer beliebigen Drehposition, des Rotors 10, über dem sich das eingelegte Pumpensegment, das in Figur 4 mit strichpunktierter Line angedeutet ist und das Bezugszeichen PS hat, von der Anschlussstelle PN zur Anschlussstelle PP der Stützfläche 30 folgend erstreckt, kann der Rotor 10 manuell gedreht werden, indem der Anwender den Rotor 10 im Freiraum 78 (siehe Figur 2) zwischen den Führungsflächen 46, 48 erfasst. Der Leitkörper 70 berührt das Pumpensegment PS in dieser Phase noch nicht.

Wenn der Rotor weitergedreht wird, gleitet die äußere Funktionsfläche F1 des Leitkörpers 70 - wie in Figur 4 in sequentiellen Positionen angedeutet - auf das Pumpensegment PS auf, und beim Weiterdrehen des Rotors 10 drückt nun die Funktionsfläche F1 des Leitkörpers 70 das Pumpensegment PS immer weiter nach unten in Richtung Pumpenbett, so dass das Pumpensegment, weil es an der Stützfläche 30 anliegt, schließlich von den Funktionsflächen F2 mittig zur Symmetrieebene ES gefangen wird. Diese Funktion setzt sich beim Überstreichen der Stützfläche 30 gleichermaßen fort, so dass schließlich das gesamte Pumpensegment zentrisch zu den Andruckrollen im Pumpenbett 7 liegt.

Wenn beim Weiterdrehen des Rotors der zum Leitkörper 70 um 180° versetzte Leitkörper (in den Figuren nicht bezeichnet), der am Einfädelvorgang nicht beteiligt war, auf das sich bereits eingefädelt Pumpensegment PS trifft, nähern sich die Leitkörper 70 und 72 in dieser Phase dem Pumpensegment derart, dass sie das Pumpensegment trichterähnlich zwischen die Funktionsflächen F2 leiten und es somit zentrisch zu den Andruckrollen 20 ausrichten. Diese Wirkungsweise ergibt sich jedes Mal, wenn im Betrieb der Peristaltikpumpe eine Andruckrolle in Funktion tritt, wodurch das Fördervolumen der Peristaltikpumpe konstant gehalten werden kann.

Im Hinblick darauf, dass die Peristaltikpumpe und damit auch die Schwinge 41 im medizinischen Bereich eingesetzt wird, und weil die mechanische Beanspruchung der Schwinge wegen der auf das Pumpensegment über die Andruckrolle auszuübenden Kräfte verhältnismäßig groß ist, besteht die Schwinge 41 neuerungsgemäß aus hochfestem Edelstahl mit hoher chemischer Beständigkeit und sie ist im Metal-Injection-Molding (MIM)- Verfahren hergestellt. Als Werkstoff kann beispielsweise ein hitzebeständiger austenitischer Chrom-Nickel-Stahl (X15CrNiSi25-21) Anwendung finden, wie er unter der Bezeichnung 1.4841 bekannt ist. Dieser Stahl ist besonders beständig gegen Oxidation, und es hat sich gezeigt, dass die im MIM-Verfahren erzielbaren Oberflächenqualitäten ausreichend sind, um die Funktionsflächen F1 und F2 ohne weitere Nachbearbeitung glatt genug zu machen, um das Pumpensegment PS sanft und ohne Abrieb in das Pumpenbett zu drücken und zentrisch zu den Andruckrollen auszurichten.

Dadurch, dass die Leitkörper 70, 72 an der Schwinge 41, und zwar jeweils an einem Schwenklagerauge 50, 52, einstückig angebracht sind, ergibt sich für die Schwinge 41 eine aus den Darstellungen ersichtliche, verhältnismäßig komplexe Form, die im MIM-Verfahren mit minimalem Materialaufwand hergestellt werden kann. Gleichzeitig kann Material dort entfallen, wo es für die Funktion nicht wichtig ist. Auf diese Weise ist es möglich, die Schwinge 41 - wie die Ansicht gemäß Figur 5 verdeutlicht - einerseits mit vielen Material-Leerstellen zu gestalten und andererseits die Abschnitte dort zu verstärken, wo eine erhöhte mechanische Festigkeit gefordert ist. Verstärkte Lageraugen 50, 52 können so dazu genutzt werden, die Streben 76 an der Schwinge 41 zur Stützung der ansonsten recht filigranen Leitkörper 70, 72 zu nutzen. Gleichzeitig kann der Freiraum zwischen den Schwenklageraugen 50, 52 vergrößert werden, um zwischen den beiden Schwenklageraugen axial einen massiveren Gelenkstift-Halteblock 39 (siehe Figur 3) des Rotor-Grundkörpers (40) einfassen. Auch die die Schwenklageraugen 50, 52 mit der Anschlagfläche 54 verbindenden Bügelarme 58, 60 sind materialsparend ausgebildet, um viel Platz für die Aufnahme der Andruckrolle zu schaffen. Die Lagerstellen 62, 64 sind dabei verstärkt, um die Drehachse 66 verdreh- und verschiebesicher mit den Bügelarmen 58, 60 verbinden zu können.

Beim Herstellungsverfahren kann der aus der Mischung von Metallpulver und Binder gespritzte Rohling schon alle geometrischen Merkmale der fertigen Schwinge 41 aufweisen. Weder beim Entfernen des in der Regel organischen Binders noch beim Sintern gehen diese geometrischen Merkmale verloren, denn die beim Sintern auftretende Schwindung erfolgt regelmäßig linear zu Enddichten von bis zu 96 %, so dass auch die Oberfläche der Schwinge mit ausreichender Qualität nach dem Sintern ausreichend gut ist, um die vorstehenden Funktionen der Schwinge bei guter Schonung des Pumpensegments zu erfüllen. Der richtige Aufmaßfaktor kann im Übrigen durch Probesinterungen exakt bestimmt werden.

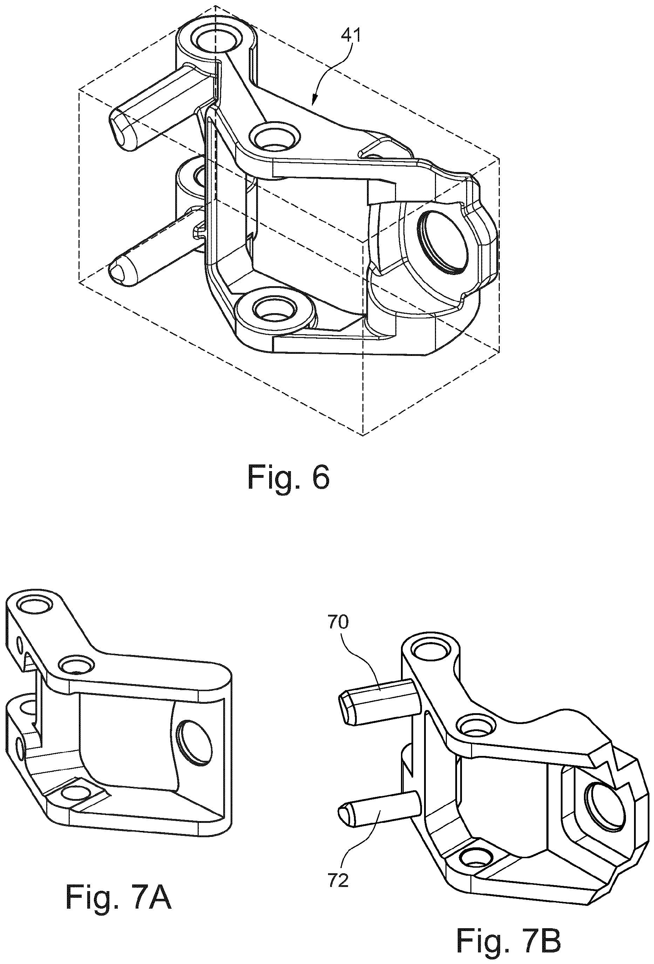

Figur 6 zeigt auf, welche Materialersparnis sich mit der neuerungsgemäßen Schwinge im Vergleich zu einer spanabhebend gefertigten Schwinge erzielbar ist. Bei spanabhebender Fertigung wird ein mit gestrichelter Linie gezeigter Metallblock von ca. 40 cm³ benötigt, von dem ca. 36 cm³ Material in komplexen Arbeitsschritten abgetragen werden müsste. Dementsprechend beträgt der Materialaufwand der oben beschriebenen Schwinge nur ca. 4 cm³, so dass eine erhebliche Rohstoffeinsparung möglich ist.

Aus der Figur 7A kann ersehen werden, wie sich eine mit vertretbarem Fertigungsaufwand spanabhebend herstellbare Schwinge von der neuerungsgemäß hergestellten und gestalteten Schwinge unterscheidet. Man erkennt, dass lediglich ein Grundkörper mit recht einfachen Flächen ohne Verstrebungen hergestellt werden kann, mit dem dann noch die Komponenten für die Einführhilfe und die Schlauchführungselemente verbunden werden müssen. Verstrebungen sind mit vertretbarem Aufwand nicht darstellbar. Als Material kommt nur ein Werkstoff in Frage, der einfach zu zerspanen ist, so dass zusätzlicher Aufwand für eine Oberflächenbehandlung erforderlich ist.

Mit der Darstellung gemäß Figur 7B wird verdeutlicht, welch zusätzlicher Materialaufwand erforderlich wird, wenn mittels eines herkömmlichen Fräsverfahrens eine Schwinge hergestellt wird, bei der Schlauchführungselemente in Form von stiftartigen Leitkörpern 70, 72 einstückig in die Schwinge integriert sind.

Demgegenüber stellt sich die neuerungsgemäße Schwinge - wie in Figur 6 gezeigt - als Bauteil dar, das trotz geringem Materialeinsatz hochfest ist, wobei eine weitere Bearbeitung der Oberflächen entfallen kann.

Selbstverständlich sind Abweichungen vom beschriebenen Ausführungsbeispiel möglich, ohne den Grundgedanken der Erfindung zu verlassen.

Die Leitkörper 70, 72 können bei Bedarf auch zusätzlich mit einem Werkstoff beschichtet sein, der abriebminimierende Gleiteigenschaften hat.

Für die Herstellung der Schwinge 41 kann auch ein anderes Pulverspritzgießverfahren angewendet werden, beispielsweise das Keramikpulverspritzgießen.

Es ist auch möglich, die Leitkörper 70, 72 als gesonderte Bauteile auszubilden, die dann mit der Schwinge 41 zu einer integralen Baueinheit verbunden werden.

Bei der vorstehend beschriebenen Peristaltikpumpe sind zwei Quetschelemente vorgesehen, die in einer relativen Position von 180° starr bzw. in einem festen Winkel von 180° zueinander angeordnet sind. Es kann jedoch auch vorgesehen sein, dass mehr als zwei Quetschelemente vorhanden sind und dass die Quetschelemente in Rotationsrichtung relativ zueinander winkelpositionierbar ausgebildet sind. Hierzu können die Quetschelemente auf Rotorarmen angeordnet sein, die gegenüber dem Rotor in Umfangsrichtung verschwenkbar ausgebildet sein können.

Die Quetschelemente müssen nicht als Quetschrollen oder Andruckrollen, die in vorteilhafter Weise materialschonend an der Fluidleitung abrollen, ausgebildet sein. Es können auch Gleitschuhe, die sich gleitend über das Schlauchsegment bewegen, vorgesehen sein.

Die Erfindung schafft somit eine Peristaltikpumpe, insbesondere zum Fördern von Fluid in einer Vorrichtung zur extrakorporalen Blutbehandlung, mit einem Pumpengehäuse, in dem ein um eine Rotorachse rotierbarer Rotor mit zumindest zwei in Umfangsrichtung zueinander versetzten Quetschelementen aufgenommen ist und welches eine sich bogenförmig um die Rotorachse erstreckende und radial von dem Rotor beabstandete Stützfläche aufweist, die zum Abstützen eines radial zwischen den Rotor und die Stützfläche einbringbaren Schlauchsegments eingerichtet ist. Der Rotor trägt das Quetschelement über eine Schwinge, die schwenkbeweglich und abgefedert am Rotor festgelegt ist. Die Schwinge trägt ihrerseits eine Einführhilfe, mit der das Schlauchsegment bei dessen Einfädelvorgang in das Pumpenbett drückbar ist, sowie Schlauchführungselemente trägt, mit denen das Schlauchsegment mittig zum Quetschelement ausrichtbar ist. Zur Bereitstellung einer Peristaltikpumpe, die sich bei erweiterter Funktionalität und Lebensdauer durch eine vereinfachte Herstellung auszeichnet, besteht die Schwinge aus hochfestem Edelstahl mit hoher chemischer Beständigkeit und sie ist im Pulverspritzgieß-, vorzugsweise im Metal-Injection-Molding (MIM)-Verfahren hergestellt.

### Bezugszeichenliste

- A: Rotorachse
- D: Drehrichtung
- PN: Niederdruck-Anschlussstelle
- PP: Hochdruck-Anschlussstelle
- PS: Pumpensegment
- ES: Symmetrieebene
- F1: Funktionsfläche
- F2: Funktionsfläche
- FR: Radialrichtung

- 5: Pumpengehäuse
- 7: Pumpenbett
- 10: Rotor
- 20: Quetschelement
- 30: Stützfläche
- 38: Gelenkstift
- 39: Gelenkstift-Halteblock
- 40: Rotor-Grundkörper
- 42: Rotorabdeckung
- 44: Taste
- 46: Führungsfläche
- 48: Führungsfläche
- 50: Lagerauge
- 52: Lagerauge
- 54: Anschlagfläche
- 56: Druckfeder
- 58: oberer Bügelarm
- 60: unterer Bügelarm
- 62: obere Lagerstelle
- 64: untere Lagerstelle
- 66: Drehachse
- 70: oberer Leitkörper
- 72: unterer Leitkörper
- 76: Strebe
- 78: Freiraum

- 305: Gehäuse
- 307: Pumpenbett
- 310: Rotor
- 320: Quetschelemente
- 321: Rollenachse
- 330: Stützfläche
- 341: Schwinge
- 336: Druckfeder
- 338: Gelenkstift
- A338: Achse
- 360: Führungsvorsprünge, Hacken
- 370: Führungsstifte

## Patentansprüche

1. Peristaltikpumpe, insbesondere zum Fördern von Fluid in einer Vorrichtung zur extrakorporalen Blutbehandlung, mit
einem Pumpengehäuse (5), in dem ein um eine Rotorachse (A) rotierbarer Rotor (10) mit zumindest zwei in Umfangsrichtung zueinander versetzten Quetschelementen (20) aufgenommen ist und welches eine sich bogenförmig um die Rotorachse (A) erstreckende und radial von dem Rotor (10) beabstandete Stützfläche (30) aufweist, die zum Abstützen eines radial zwischen den Rotor (10) und die Stützfläche (30) einbringbaren Schlauchsegments (PS) eingerichtet ist, wobei der Rotor (10)
für das Quetschelement (20) eine Schwinge (41), die schwenkbeweglich und abgefedert am Rotor (10) festgelegt ist, eine Einführhilfe (70, F1), mit der das Schlauchsegment (PS) bei dessen Einfädelvorgang in das Pumpenbett (7) drückbar ist, und Schlauchführungselemente (70, 72) trägt, mit denen das Schlauchsegment (PS) mittig zum Quetschelement (20) ausrichtbar ist, **dadurch gekennzeichnet, dass**
die Schwinge (41) aus hochfestem Edelstahl mit hoher chemischer Beständigkeit besteht und im Pulverspritzgieß-, vorzugsweise im Metal-Injection-Molding (MIM)-Verfahren hergestellt ist.

2. Peristaltikpumpe nach Anspruch 1, **dadurch gekennzeichnet, dass** die Funktionsoberflächen der Schwinge nach dem Sinterprozess keiner weiteren die Oberfläche glättenden Behandlung unterzogen sind.

3. Peristaltikpumpe nach Anspruch 1 oder 2, **dadurch gekennzeichnet, dass** sowohl die Einführhilfe (70, F1) als auch die Schlauchführungselemente (70, 72, F2) dem Quetschelement (20) in Drehrichtung (D) des Rotors (10) benachbart in die Schwinge (41) integriert sind.

4. Peristaltikpumpe nach einem der Ansprüche 1 bis 3, **dadurch gekennzeichnet, dass** die Schwinge (41) am Rotor (10) mittels eines Schwenklagers (38) angebracht ist, welches zum Quetschelement (20) in Drehrichtung versetzt angeordnet ist, wobei die Einführhilfe (70, F1) und die Schlauchführungselemente (70, 72, F2) einstückig am Schwenklager (38) sitzen.

5. Peristaltikpumpe nach Anspruch 4, **dadurch gekennzeichnet, dass** die Einführhilfe (70, F1) von einem stiftartigen Leitkörper (70) gebildet ist, der sich vom Schwenklager (38) im Wesentlichen in radialer Richtung von der Rotorachse (A) weg erstreckt und der im Querschnitt betrachtet im Wesentlichen eine abgerundete, in Drehrichtung (D) weisende Keilform hat, die zum Pumpenbett (7) unter einem spitzen Winkel (WA) angestellt ist.

6. Peristaltikpumpe nach Anspruch 5, **dadurch gekennzeichnet, dass** der Leitkörper (70) zwei Funktionsflächen (F1, F2) aufweist, von denen die in Drehrichtung (D) des Rotors (10) vorlaufende, einer Mittelebene (ES) abgewandte äußere Funktionsfläche (F1) eine abgerundete Keilfläche ausbildet und die der Mittelebene (ES) zugewandte innere Funktionsfläche (F2) von einer sich an die Keilfläche vorzugsweise stufenlos anschließenden Zylindersegmentfläche gebildet ist.

7. Peristaltikpumpe nach Anspruch 4 oder 5, **dadurch gekennzeichnet, dass** der Querschnitt des Leitkörpers (70) eine Mittelachse (SA70) hat, wobei der Leitkörper (70) vorzugsweise zusätzlich mittels einer der Mittelachse (SA70) folgenden Strebe (76) an der Schwinge (41) abgestützt ist.

8. Peristaltikpumpe nach einem der Ansprüche 3 bis 7, **dadurch gekennzeichnet, dass** der Leitkörper (70) an einem Schwenklagerauge (50) der Schwinge (41) vorzugsweise einstückig angebracht ist.

9. Peristaltikpumpe nach Anspruch 8, **dadurch gekennzeichnet, dass** die Schwinge (41) an ihrem dem Schwenklagerauge (50, 52) abgewandten Endabschnitt eine Anschlagfläche (54) für eine Druckfeder (56) ausbildet, wobei das Quetschelement (20) zwischen Schwenklagerauge (50, 52) und Anschlagfläche (54) angeordnet, beispielsweise drehbar gelagert ist.

10. Peristaltikpumpe nach einem der Ansprüche 3 bis 9, **dadurch gekennzeichnet, dass** dem die Einführhilfe (70, F1) ausbildenden ersten Leitkörper (70) ein weiterer, zweiter Leitkörper (72) zugeordnet ist, der bezüglich einer parallel zum Pumpenbett (7) liegenden Horizontalebene (ES) symmetrisch zum ersten Leitkörper (70) ausgebildet und orientiert ist, so dass die sich zugewandten Zylindersegmentflächen (F2) der beiden Leitkörper (70, 72) die Funktionsflächen der Schlauchführungselemente (70, 72, F2) ausbilden.

11. Peristaltikpumpe nach Anspruch 10, **dadurch gekennzeichnet, dass** der zweite Leitkörper (72) vorzugsweise einstückig an einem weiteren Schwenklagerauge (52) angebracht ist, und dass die beiden Schwenklageraugen (50, 52) axial einen Gelenkstift-Halteblock (39) des Rotor-Grundkörpers (40) einfassen.

12. Peristaltikpumpe nach Anspruch 10, **dadurch gekennzeichnet, dass** die Horizontalebene (ES) eine Symmetrieebene der Schwinge (41) ist.

13. Peristaltikpumpe nach einem der Ansprüche 1 bis 11, **dadurch gekennzeichnet, dass** zumindest die Einführhilfe (70, F1) und/oder die Schlauchführungselemente (70, 72, F2) von einem Werkstoff gebildet und/oder beschichtet sind, der abriebminimierende Gleiteigenschaften hat.

14. Für eine Peristaltikumpe hergerichtete Schwinge mit den Merkmalen gemäß einem der Ansprüche 1 bis 12.
